# EUROPÄISCHE PATENTSCHRIFT

(11) **EP 2 257 532 B1**
(45) Veröffentlichungstag und Bekanntmachung des Hinweises auf die Patenterteilung: **29.05.2013**
(21) Anmeldenummer: 09719701.6
(22) Anmeldetag: 26.02.2009
(51) Int. Cl.: C07D 231/14

(54) **VERFAHREN ZUR REGIOSELEKTIVEN SYNTHESE VON L-ALKVL-3-HALOALKYL-PVRAZOL-4-CARBONSÄURE-DERIVATEN**
METHOD FOR REGIOSELECTIVE SYNTHESIS OF 1-ALKYL-3-HALOALKYL-1-PYRAZOL-4-CARBONIC ACID DERIVATIVES
PROCÉDÉ DE SYNTHÈSE RÉGIOSÉLECTIVE DE DÉRIVÉS D'ACIDES CARBONIQUES 1-ALCYL-3-HALOALCYL-PYRAZOL-4

(30) Priorität: 10.03.2008 EP 08152532
(43) Veröffentlichungstag der Anmeldung: 08.12.2010
(73) Patentinhaber: BAYER CROPSCIENCE AG, 40789 Monheim (DE)
(72) Erfinder: PAZENOK, Sergii, 42699 Solingen (DE); NEEFF, Arnd, 51399 Burscheid (DE); LUI, Norbert, 51519 Odenthal (DE)
(86) Internationale Anmeldenummer: PCT/EP2009/001354
(87) Internationale Veröffentlichungsnummer: WO 2009/112157

(56) Entgegenhaltungen:
- EP-A- 1 854 788
- WO-A-2005/123690

## Beschreibung

Die vorliegende Erfindung betrifft ein Verfahren zur regioselektiven Synthese von 1-Alkyl-3-haloalkyl-pyrazol-4-carbonsäure-Derivaten durch Cyclisierung von 2,3-disubstituierten Acrylsäure-Derivaten mit Hydrazinen, in Gegenwart von Carbonylverbindungen.

2-Dihalogenacyl-3-dialkylamino-acrylsäureester der Formel II (Y=COOAlk, Z=O) sind wertvolle Zwischenprodukte für die Herstellung von dihalogenmethyl-substituierten Pyrazolylcarbonsäure-Derivaten, die als Vorstufen von fungiziden Wirkstoffen verwendet werden können (vgl. WO 03/070705).

Pyrazolcarbonsäure-Derivate werden üblicherweise hergestellt, indem man Acrylsäure-Derivate, die zwei Abgangsgruppen (Z und A) aufweisen, mit Hydrazinen umsetzt.

Bei der Umsetzung mit den Monoalkylhydrazinen erhält man vorwiegend 1-Alkylpyrazole. Allerdings erfolgt die Cyclisierung oftmals nicht regioselektiv. Folglich werden, in Abhängigkeit von Substrat und Reaktionsbedingungen, die unerwünschten 5-Alkylpyrazole in Mengen zwischen 10 und 80 % gebildet (siehe Schema 1).

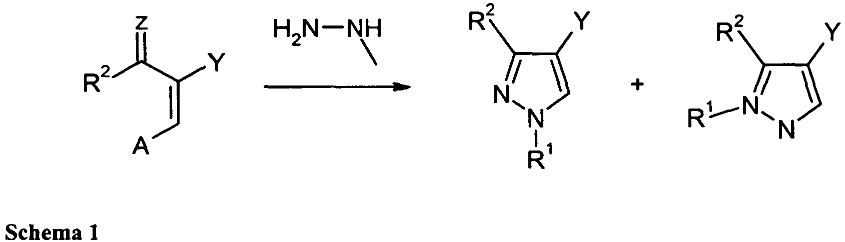

Auch die Synthese von 1-Alkylpyrazolcarbonsäure-Derivaten durch Alkylierung von in 1-Position unsubstituerten Pyrazolderivaten verläuft oftmals unter Bildung beider Regioisomere (siehe Schema 2).

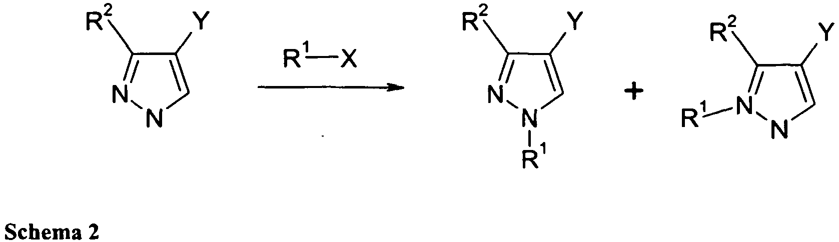

Ein alternativer Weg zur Herstellung von Fluorhaloalkylpyrazolencarbonsäuren ist die Cyclisierung von z. B 4,4-Dichloro-2-[(dimethylamino)methylidene]-3-oxobutanoat mit Alkylhydrazinen, gefolgt von einem Halogenaustausch.

WO 2005/042468 offenbart ein Verfahren zum Herstellen von 2-Dihalogenacyl-3-amino-acrylsäureestem durch Umsetzung von Säurehalogeniden mit Dialkylaminoacrylsäureestern und deren anschließende Cyclisierung mit Alkylhydrazinen

Die bislang unveröffentlichte Europäische Patentanmeldung Nr. 07117232.4 beschreibt ein Verfahren zur Herstellung von HCl-freien 2-Dihalogenacyl-3-amino-acrylsäureestem durch Umsetzung von Säurefluoriden mit Dialkylaminoacrylsäure-Derivaten. Das Verfahren kann in Abwesenheit einer Base durchgeführt werden, wodurch die Abtrennung von Halogenid-Salzen entfällt.

WO 2008/022777 beschreibt ein Verfahren zum Herstellen von 3-Dihalomethyl-pyrazol-4-carbonsäure-Derivaten durch Umsetzung von α,α-Fluoraminen in Gegenwart von Lewissäuren mit Acrylsäure-Derivaten und deren anschließender Umsetzung mit Alkylhydrazinen.

WO 2006/090778 offenbart ein Verfahren zur Herstellung von 1-Methyl-3-difluormethyl-pyrazolcarbonsäureestem durch Cyclisierung von 2-Alkoxymethylenfluoracylacetat mit Methylhydrazin in Anwesenheit von Wasser und einer Base. Die Umsetzung wurde in Gegenwart von NaOH oder KOH durchgeführt obwohl unter diesen stark alkalischen Bedingungen teilweise Verseifung von COOEt-Gruppe des Pyrazolrings auch stattfindet.

Die zuvor beschriebenen Verfahren weisen jedoch alle den Nachteil auf, dass die Cyclisierung, selbst bei niedrigen Temperaturen, nur mit unbefriedigender Regioselektivität erfolgt.

Im Lichte des zuvor beschrieben Standes der Technik, liegt der vorliegenden Erfindung die Aufgabe zugrunde, ein Verfahren zur Verfügung zu stellen, dass die zuvor genannten Nachteile nicht aufweist und somit einen regioselektiven Zugang zu 1-Alkyl-3-haloalkyl-pyrazol-4-carbonsäure-Derivaten in hohen Ausbeuten gewährt.

Die zuvor beschriebene Aufgabe wurde gelöst durch ein Verfahren zum Herstellen von 1-Alkyl-3-haloalkyl-pyrazol-4-carbonsäure-Derivaten der Formel (I) 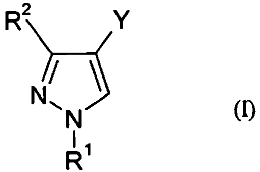 in welcher
- R¹: ausgewählt ist aus C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können;
- R²: ausgewählt ist aus C₁-C₄-Alkylgruppen, die mit einem, zwei oder drei Halogenatomen, ausgewählt aus F, Cl und Br oder einer CF₃-Gruppe substituiert sein können;
- Y: ausgewählt ist aus der Gruppe bestehend aus (C=O)OR³, CN und (C=O)NR⁴R⁵, wobei R³, R⁴ und R⁵ unabhängig voneinander ausgewählt sind aus Wasserstoff, C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können;
oder wobei R⁴ und R⁵ gemeinsam mit dem N-Atom an das sie geknüpft sind einen fünf oder sechsgliedrigen Ring bilden können;
umfassend die Umsetzung eines 2-acylierten Acrylsäure-Derivats der Formel (II), 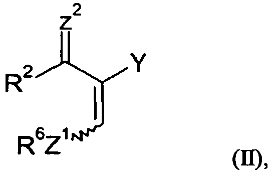 in welcher
- Z¹ und Z²: unabhängig voneinander ausgewählt sind aus O und S,
- R⁶: ausgewählt ist aus C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können;
mit einem N-Alkyl-Hydrazin der Formel (III) 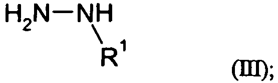 in Gegenwart einer Verbindung der Formel (IV) 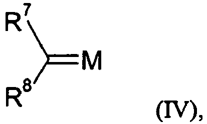 in welcher
- R⁷, R⁸: unabhängig voneinander ausgewählt sind aus H, C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', - (C=O)R', -CN and -CONR₂', wobei R' Wasserstoff oder eine C₁₋₁₂-AlkylGruppe ist, substituiert sein können; und
- M: ausgewählt ist aus O, S, Se, NH, NR' und OR', wobei R' die zuvor definierten Bedeutungen hat.

Überraschenderweise lassen sich die 1-Alkyl-3-haloalkyl-pyrazol-4-carbonsäure-Derivate der Formel (I) unter den erfindungsgemäßen Bedingungen mit guten Ausbeuten, Regioselektivitäten und in hoher Reinheit herstellen, womit das in Schema 3 dargestellte, erfindungsgemäße Verfahren die oben genannten Nachteile der im Stand der Technik vorbeschriebenen Herstellungsverfahren überwindet.

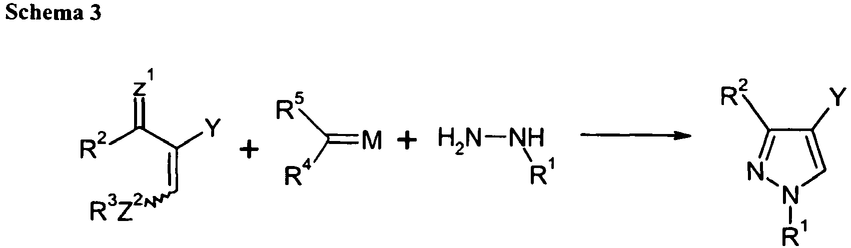

### Allgemeine Definitionen

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen sind beispielsweise ausgewählt aus Trifluormethyl (CF₃), Difluormethyl (CHF₂), CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen, die optional ein, zwei oder mehrere Heteroatome, die ausgewählt sind aus O, N, P und S aufweisen können. Außerdem können die erfindungsgemäßen Alkyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Cycloalkyl-Gruppen sind im Zusammenhang mit der vorliegenden Erfindung, soweit nicht abweichend definiert, monocyclische, gesättigte Kohlenwasserstoffgruppen mit 3 bis 8 Kohlenstoffringgliedern, die optional ein, zwei oder mehrere Heteroatome, die ausgewählt sind aus O, N, P und S aufweisen können. Außerdem können die erfindungsgemäßen Cycloalkyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl-(-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Im Einzelnen umfasst diese Definition beispielsweise die Bedeutungen Cyclopropyl, Cyclobutyl, Cyclopentyl und Cyclohexyl.

Die Definition C₁-C₁₂-Alkyl umfasst den größten hierin definierten Bereich für einen AlkylGruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Alkenyl-Gruppen sind im Zusammenhang mit der vorliegenden Erfindung, soweit nicht abweichend definiert, lineare oder verzweigte Kohlenwasserstoff-Gruppen, die wenigstens eine einfache Unsättigung (Doppelbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkenyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl-(-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₂-C₁₂-Alkenyl umfasst den größten hierin definierten Bereich für einen Alkenyl-Gruppe. Im Einzelnen umfasst diese Definition beispielsweise die Bedeutungen Vinyl; Allyl (2-Propenyl), Isopropenyl (1-Methylethenyl); But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl; Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl; Buta-1,3-dienyl, Penta-1,3-dienyl.

Cycloalkenyl-Gruppen sind im Zusammenhang mit der vorliegenden Erfindung, soweit nicht abweichend definiert, monocyclische, nicht aromatische Kohlenwasserstoffgruppen mit 3 bis 8 Kohlenstoffringgliedern mit mindestens einer Doppelbindung, die optional ein, zwei oder mehrere Heteroatome, die ausgewählt sind aus O, N, P und S aufweisen können. Außerdem können die erfindungsgemäßen Cycloalkenyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Im Einzelnen umfasst diese Definition beispielsweise die Bedeutungen Cyclopenten-1-yl, Cyclohexen-1-yl, Cyclohepta-1,3-dien-1-yl.

Alkinyl-Gruppen sind im Zusammenhang mit der vorliegenden Erfindung, soweit nicht abweichend definiert, lineare, verzweigte oder ringförmige Kohlenwasserstoff-Gruppen, die wenigstens eine zweifache Unsättigung (Dreifachbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkinyl-Gruppen optional durch weitere Gruppen substituiert sein, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine lineare, verzweigte oder cyclische C₁₋₁₂-Alkyl-Gruppe ist, die ein oder mehrere Heteroatome, ausgewählt aus N, O, P und S, aufweisen kann.

Die Definition C₂-C₁₂-Alkinyl umfasst den größten hierin definierten Bereich für einen Alkinyl-Gruppe. Im Einzelnen umfasst diese Definition beispielsweise die Bedeutungen Ethinyl (Acetylenyl); Prop-1-inyl und Prop-2-inyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S aufweisen können und optional durch weitere Gruppen substituiert sein können, die ausgewählt sind aus -R', Halogen- (-X), Alkoxy-(-OR'), Thioether- oder Mercapto- (-SR'), Amino- (-NR'₂), Silyl- (-SiR'₃), Carboxyl-(-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₅₋₁₈-Aryl umfasst den größten hierin definierten Bereich für eine Aryl-Gruppe mit 5 bis 18 Gerüst-Atomen, wobei die C-Atome gegen Heteroatome ausgetauscht sein können. Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Die Alkyl-, Alkenyl-, Alkinyl-, Aryl,- Alkaryl- und Aralkylgruppen können zudem ein oder mehrere Heteroatome aufweisen, die - soweit nicht abweichend definiert - ausgewählt sind aus N, O, P und S. Die Heteroatome ersetzen dabei die bezifferten Kohlenstoffatome.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

Die gemäß dem erfindungsgemäßen Verfahren erhältlichen 1-Alkyl-3-haloalkyl-pyrazol-4-carbonsäure-Derivaten sind Verbindungen der Formel (I)

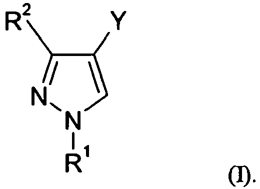

Die Reste in Formel (I) haben erfindungsgemäß die folgenden Bedeutungen:
- R¹: ist ausgewählt aus C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können;
- R²: ist ausgewählt aus C₁-C₄-Alkylgruppen, die mit einem, zwei oder drei Halogenatomen, ausgewählt aus F, Cl und Br oder einer CF₃-Gruppe substituiert sein können;
- Y: ist ausgewählt aus der Gruppe bestehend aus (C=O)OR³, CN und (C=O)NR⁴R⁵, wobei R³, R⁴ und R⁵ unabhängig voneinander ausgewählt sind aus C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können;
oder wobei R⁴ und R⁵ gemeinsam mit dem N-Atom an das sie geknüpft sind einen fünf oder sechsgliedrigen Ring bilden können.

In einer *bevorzugten* Ausführungsform der vorliegenden Erfindung haben die Reste in Formel (I) die folgenden Bedeutungen:
- R¹: ist ausgewählt aus Methyl, Ethyl, n-Propyl oder Isopropyl,
- R²: ist ausgewählt aus Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl; 1,2,2,2-Tetrafluorethyl.
- Y: ist ausgewählt aus der Gruppe bestehend aus (C=O)OR³, CN und (C=O)NR⁴R^{5,} wobei R³, R⁴ und R⁵ unabhängig voneinander ausgewählt sind aus Methyl, Ethyl, n-Propyl oder Isopropyl,

In einer besonders bevorzugten Ausführungsform der vorliegenden Erfindung haben die Reste in Formel (I) die folgenden Bedeutungen:
- R¹: ist Methyl,
- R²: ist ausgewählt aus Trifluormethyl oder Difluormethyl
- Y: ist ausgewählt aus der Gruppe bestehend aus (C=O)OR³, wobei R³ Methyl oder Ethyl ist.

Die Durchführung des erfindungsgemäßen Verfahrens erfolgt vorzugsweise innerhalb eines Temperaturbereichs von -20°C bis +150°C, besonders bevorzugt bei Temperaturen von -10°C bis +70°C.

Das erfindungsgemäße Verfahren wird im Allgemeinen unter Normaldruck durchgeführt. Alternativ ist es jedoch auch möglich, im Vakuum zu arbeiten, um die leicht flüchtigen Dialkylamine zu entfernen.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrens setzt man 1 Mol des Acrylsäure-Derivats der Formel (II) mit 0.5 Mol bis 3 Mol, vorzugsweise 0.5 Mol bis 1.5 Mol, besonders bevorzugt mit der äquimolaren Menge des Hydrazins der Formel (III) und mit 0.5 Mol bis 50 Mol, vorzugsweise 0.5 Mol bis 20 Mol, besonders bevorzugt mit 1 bis 2 Mol der Verbindung der Formel (IV) um.

Vorzugsweise wird das im Lösungsmittel gelöste Hydrazin der Formel (III) zusammen mit Verbindung der Formel (IV) vorgelegt und das Acrylsäure-Derivat der Formel (II) zugegeben. Die umgekehrte Reihenfolge ist jedoch auch möglich. Idealerweise wird die Reaktion direkt in Überschuss von Verbindung der Formel (IV) zum Beispiel in Acetone oder Pinakolon durchgeführt. Vor der Isolierung des Produktes wird zu dem Reaktionsgemisch die Säure zugegeben. Geeignete Säuren sind ausgewählt aus der Gruppe bestehend aus HCl, H₂SO₄, CF₃COOH, CF₃SO₃H, CH₃COOH, besonders bevorzugt sind HCl und H₂SO₄.

Die Acrylsäure-Derivate der Formel (II) sind erhältlich nach den zuvor, im Zusammenhang mit dem Stand der Technik, beschriebenen Verfahren.

Im Zusammenhang mit der vorliegenden Erfindung werden vorzugsweise 2-acylierte Acrylsäure-Derivate der Formel (II) verwendet, die ausgewählt sind aus der Gruppe bestehend aus Ethyl-(2-ethoxymethylen)-4,4-difluormethylacetoacetat, Ethyl-(2-ethoxymethylen)-4,4,4-trifluormethylacetoacetat , Ethyl-(2-ethoxymethylen)-4,4,4-trifluormethylacetonitril.

Die Monoalkyl-Hydrazine der Formel (III) sind im Zusammenhang mit der vorliegenden Erfindung vorzugsweise ausgewählt aus der Gruppe bestehend aus Monomethylhydrazin, Monoethylhydrazin, Monopropylhydrazin, Phenylhydrazin.

Die Verbindungen der Formel (IV) sind im Zusammenhang mit der vorliegenden Erfindung vorzugsweise Carbonylverbindungen, besonders bevorzugt sind diese ausgewählt aus der Gruppe bestehend aus Aceton, Pinakolon, Benzaldehyd, Benzophenon, Cyclohexanon, Methylethylketon, ganz besonders bevorzug sind Aceton, Pinacolon und Benzaldehyd.

Ein weiterer Vorteil des erfindungsgemäßen Verfahrens, ist die Tatsache, dass zur Herstellung von Pyrazolen der Formel 1 wässrige Methylhydrazinlösungen verwendet werden können und nicht zwangsläufig das explosive, auch als Raketentreibstoff verwendete, konzentrierte Methylhydrazin eingesetzt werden muss.

Die Reaktion kann in Substanz oder in einem Lösungsmittel durchgeführt werden. Vorzugsweise wird die Reaktion in einem Lösungsmittel durchgeführt. Geeignete Lösungsmittel sind beispielsweise ausgewählt aus der Gruppe bestehend aus aliphatischen und aromatischen Kohlenwasserstoffen, wie z.B. Wasser, Alkohole (Methanol, Ethanol, Isopropanol), n-Hexan, Benzol oder Toluol, die durch Fluor- und Chloratome substituiert sein können, wie Methylenchlorid, Dichlorethan, Fluorbenzol, Chlorbenzol oder Dichlorbenzol; Ethern, wie z.B. Diethylether, Diphenylether Methyl-tert-butylether, Isopropylethylether, Dioxan, Diglym, Dimethylglycol, Dimethoxyethane (DME) oder THF; Nitrilen wie Methylnitril, Butylnitril oder Phenylnitril; Amide wir Dimethylformamid (DMF) oder N-methlypyrollidon (NMP) oder Mischungen solcher Lösungsmittel geeignet, wobei Acetonitril, Dichlormethan, THF, DME und Ethylacetate, Acetone, Wasser, Ethanol besonders bevorzugt sind.

Nach beendeter Reaktion wird beispielsweise die Lösemitteln entfernt und das Produkt durch Filtration isoliert oder zunächst mit Wasser extrahiert, die organische Phase abgetrennt und das Lösungsmittel durch Destillation entfernt.

Das erfindungsgemäße Verfahren soll in den nachstehenden Beispielen weiter beschrieben werden. Die Beispiele sind jedoch nicht in einschränkender Weise zu interpretieren.

### Herstellungsbeispiele

### Beispiel 1: Ethyl-3-(difluoromethyl)-1-methyl-1H-pyrazol-4-carboxylat

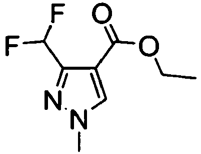

100 ml Aceton und 12 g von Methylhydrazin wurden bei 10°C zusammengemischt und das Gemisch 1 h bei RT nachgerührt.

5.8 g (25 mmol) Ethyl 2-(Ethoxymethyliden)-4,4-difluoro-3-oxobutanoat wurde zugegeben und das Gemisch 3 h bei RT nachgerührt und dann mit 1 ml 10 % HCl versetzt. Das GC zeigte nur ein Isomer. Das Gemisch wurde eingeengt und das Produkt mit kaltem Wasser gewaschen. Ausbeute 48.3 g (94 %).
**¹⁹F-NMR** (CDCl₃): *δ* =-117,2 (d) ppm.
**¹H-NMR** (CDCl₃): *δ*= 1,35 (t, 3H); 3,96 (s, 3H); 4,31 (kw, 2H); 7,10 (t, 1H), 8,15 (s, 1H) ppm.

### Beispiel 2: Ethyl 3-(chlorfluormethyl)-1-methyl-1H-pyrazol-4-carboxylat

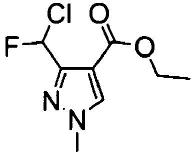

Abweichend von Beispiel 1 wird 2-(Ethoxymethyliden)-4-chlor-4,4-difluor-3-oxobulanoate eingesetzt.
**¹⁹F-NMR** (CDCl₃): δ=-133,8 (d, J=47,5) ppm.

## Patentansprüche

1. Verfahren zum Herstellen von 1-Alkyl-3-haloalkyl-pyrazol-4-carbonsäure-Derivaten der Formel (I) 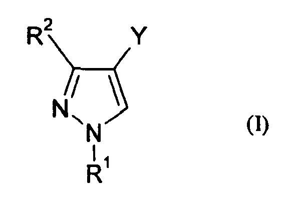 in welcher
R¹ ausgewählt ist aus C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -Hal, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können;
R² ausgewählt ist aus C₁-C₄-Alkylgruppen, die mit einem, zwei oder drei Halogenatomen, ausgewählt aus F, Cl und Br oder einer CF₃-Gruppe substituiert sein können;
Y ausgewählt ist aus der Gruppe bestehend aus (C=O)OR³, CN und (C=O)NR⁴R⁵, wobei R³, R⁴ und R⁵ unabhängig voneinander ausgewählt sind aus Wasserstoff, C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -Hal, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können;
oder wobei R⁴ und R⁵ gemeinsam mit dem N-Atom an das sie geknüpft sind einen fünf oder sechsgliedrigen Ring bilden können;
umfassend die Umsetzung eines 2-acylierten Acrylsäure-Derivats der Formel (II), 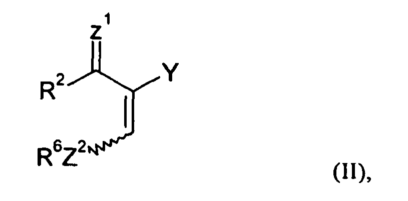 in welcher
Z¹ und Z² unabhängig voneinander ausgewählt sind aus O und S;
R⁶ ausgewählt ist aus C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus - R⁷, -Hal, -OR', -SR', -NR'₂, -SiR'₃, -COOR', -(C=O)R', -CN and -CONR₂', wobei R' Wasserstoff oder eine C₁₋₁₂-AlkylGruppe ist, substituiert sein können;
R² und Y die zuvor beschriebenen Bedeutungen haben;
mit einem N-Alkyl-Hydrazin der Formel (III) 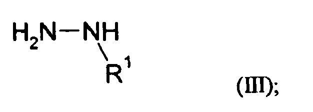 in welcher
R¹ die zuvor beschriebene Bedeutung hat;
in Gegenwart von einer Verbindung der Formel (IV) 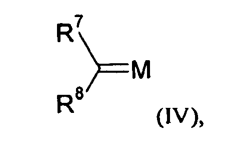 in welcher
R⁷, R⁸ unabhängig voneinander ausgewählt sind aus H, C₁₋₁₂-Alkyl-, C₃₋₈-Cycloalkyl-, C₆₋₁₈-Aryl-, C₇₋₁₉-Arylalkyl- oder C₇₋₁₉-Alkylarylresten;
M ausgewählt ist aus O, S, Se, NH, NR' und OR', wobei R' die zuvor definierten Bedeutungen hat.

2. Verfahren gemäß Anspruch 1, **dadurch gekennzeichnet, dass** das 2-acylierte Acrylsäure-Derivat der Formel (II) ausgewählt ist aus der Gruppe bestehend aus Ethyl-(2-ethoxymethylen)-4,4-difluormethylacetoacetat, Ethyl-(2-ethoxymethylen)-4,4,4-trifluormethylacetoacetat, Ethyl-(2-ethoxymethylen)-4,4,4-trifluormethylacetonitril.

3. Verfahren gemäß einem der Ansprüche 1 bis 2, **dadurch gekennzeichnet, dass** das N-Alkyl-Hydrazine der Formel (III) ausgewählt ist aus der Gruppe bestehend aus Monomethylhydrazin, Monoethylhydrazin, Phenylhydrazin.

4. Verfahren gemäß einem der Ansprüche 1 bis 3, **dadurch gekennzeichnet, dass** die Verbindung der Formel (IV) ausgewählt ist aus der Gruppe bestehend aus Aceton, Pinakolon, Benzaldehyd, Benzophenon.

5. Verfahren gemäß einem der Ansprüche 1 bis 4, **dadurch gekennzeichnet, dass** als Acrylsäure-Derivat der Formel (II) Ethyl-(2-ethoxymethylen)-4,4-difluormethyl-acetoacetat, als Hydrazin der Formel (III) Methylhydrazin und als Verbindung der Formel (IV) Aceton verwendet wird.

## Claims

1. Process for preparing 1-alkyl-3-haloalkylpyrazole-4-carboxylic acid derivatives of the formula (I) 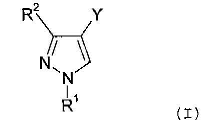 in which
R¹ is selected from the group consisting of C₁₋₁₂-alkyl, C₃₋₈-cycloalkyl, C₂₋₁₂-alkenyl, C₂₋₁₂-alkynyl, C₆₋₈-aryl, C₇₋₁₉-arylalkyl and C₇₋₁₉-alkylaryl groups, each of which may be substituted by one or more groups selected from the group consisting of - R', -Hal, -OR', -SR', -NR'₂, -SiR'₃, -COOR', -(C=O)R', -CN and -CONR₂', where R' is hydrogen or a C₁₋₁₂-alkyl group;
R² is selected from C₁-C₄-alkyl groups which may be substituted by one, two or three halogen atoms selected from the group consisting of F, Cl and Br or by a CF₃ group;
Y is selected from the group consisting of (C=O)OR³, CN and (C=O)NR⁴R⁵, where R³, R⁴ and R⁵ independently of one another are selected from the group consisting of hydrogen, C₁₋₁₂-alkyl, C₃₋₈-cycloalkyl, C₂₋₁₂-alkenyl, C₂₋₁₂-alkynyl, C₆₋₈-aryl, C₇₋₁₉-arylalkyl and C₇₋₁₉-alkylaryl groups, each of which may be substituted by one or more groups selected from the group consisting of -R', -Hal, -OR', -SR', -NR'₂, -SiR'₃, -COOR', -(C=O)R', -CN and -CONR₂', where R' is hydrogen or a C₁₋₁₂-alkyl groups;
or where R⁴ and R⁵ together with the nitrogen atom to which they are attached may form a 5- or 6-membered ring;
comprising the reaction of a 2-acylated acrylic acid derivative of the formula (II), 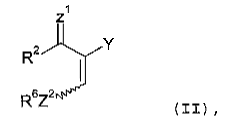 in which
Z¹ and Z² independently of one another are selected from the group consisting of O and S;
R⁶ is selected from the group consisting of C₁₋₁₂-alkyl, C₃₋₈-cycloalkyl, C₂₋₁₂-alkenyl, C₂₋₁₂-alkynyl, C₆₋₈-aryl, C₇₋₁₉-arylalkyl and C₇₋₁₉-alkylaryl groups, each of which may be substituted by one or more groups selected from the group consisting of -R', -Hal, -OR', -SR', -NR'₂, -SiR'₃, -COOR', -(C=O)R', -CN and -CONR₂', where R' is hydrogen or a C₁₋₁₂-alkyl group;
R² and Y have the meanings described above;
with an N-alkylhydrazine of the formula (III) 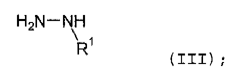 in which
R¹ has the meaning described above;
in the presence of a compound of the formula (IV) 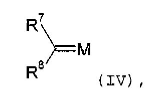 in which
R⁷, R⁸ independently of one another are selected from the group consisting of H, C₁₋₁₂-alkyl, C₃₋₈-cycloalkyl, C₆₋₁₀-aryl, C₇₋₁₅-arylalkyl and C₇₋₁₉-alkylaryl radicals;
M is selected from the group consisting of O, S, Se, NH, NR' and OR', where R' has the meanings defined above.

2. Process according to Claim 1, **characterized in that** the 2-acylated acrylic acid derivative of the formula (II) is selected from the group consisting of ethyl (2-ethoxymethylene)-4,4-difluoromethylacetoacetate, ethyl-(2-ethoxymethylene)-4,4,4-trifluoromethylacetoacetate, ethyl-(2-ethoxymethylene)-4,4,4-trifluoromethyl-acetonitrile.

3. Process according to Claim 1 or 2, **characterized in that** the N-alkylhydrazine of the formula (III) is selected from the group consisting of monomethylhydrazine, monoethylhydrazine, phenylhydrazine.

4. Process according to any of Claims 1 to 3, **characterized in that** the compound of the formula (IV) is selected from the group consisting of acetone, pinacolone, benzaldehyde, benzophenone.

5. Process according to any of Claims 1 to 4, **characterized in that** the acrylic acid derivative of the formula (II) used is ethyl (2-ethoxymethylene)-4,4-difluoromethylacetoacetate, the hydrazine of the formula (III) used is methylhydrazine and the compound of the formula (IV) used is acetone.

## Revendications

1. Procédé pour la préparation de dérivés d'acides 1-alkyl-3-halogénoalkyl-pyrazole-4-carboxyliques de formule (I) 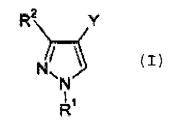 dans laquelle
R¹ est choisi parmi des groupes alkyle en C₁-C₁₂, cycloalkyle en C₃-C₈, alcényle en C₂-C₁₂, alcynyle en C₂-C₁₂, aryle en C₆-C₈, arylalkyle en C₇-C₁₉ ou alkylaryle en C₇-C₁₉, qui peuvent être substitués chacun par un ou plusieurs groupes qui sont choisis dans l'ensemble constitué par -R', -Hal, -OR', -SR', -NR'₂, -SiR'₃, -COOR', -(C=O)R', -CN et -CONR₂', R' étant un atome d'hydrogène ou un groupe allyle en C₁-C₁₂ ;
R² est choisi parmi des groupes alkyle en C₁-C₄, qui peuvent être substitués par un, deux ou trois atomes d'halogène, choisis parmi F, Cl et Br ou un groupe CF₃ ;
Y est choisi dans l'ensemble constitué par (C=O)OR³, CN et (C=O)NR⁴R⁵, R³, R⁴ et R⁵ étant choisis chacun indépendamment parmi un atome d'hydrogène, des groupes alkyle en C₁-C₁₂, cycloalkyle en C₃-C₈, alcényle en C₂-C₁₂, alcynyle en C₂-C₁₂, aryle en C₆-C₈, arylalkyle en C₇-C₁₉ ou alkylaryle en C₇-C₁₉, qui peuvent être substitués chacun par un ou plusieurs groupes qui sont choisis dans l'ensemble constitué par -R', -Hal, -OR', -SR', -NR'₂, -SiR'₃, -COOR', -(C=O)R', -CN et -CONR₂', R' étant un atome d'hydrogène ou un groupe alkyle en C₁-C₁₂ ; ou R⁴ et R⁵ pouvant former ensemble avec l'atome d'azote auquel ils sont liés un cycle à cinq ou six chaînons ;
comprenant la mise en réaction d'un dérivé d'acide acrylique 2-acylé de formule (II), 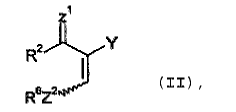 dans laquelle
Z¹ et Z² sont choisis, indépendamment l'un de l'autre, parmi O et S ;
R⁶ est choisi parmi des groupes alkyle en C₁-C₁₂, cycloalkyle en C₃-C₈, alcényle en C₂-C₁₂, alcynyle en C₂-C₁₂, aryle en C₆-C₈, arylalkyle en C₇-C₁₉ ou alkylaryle en C₇-C₁₉, qui peuvent être substitués chacun par un ou plusieurs groupes qui sont choisis dans l'ensemble constitué par -R', -Hal, -OR', -SR', -NR'₂, -SiR'₃, -COOR', -(C=O)R', -CN et -CONR₂', R' étant un atome d'hydrogène ou un groupe alkyle en C₁-C₁₂ ;
R² et Y ont les significations indiquées précédemment ;
avec une N-alkyl-hydrazine de formule (III) 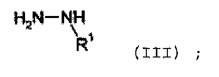 dans laquelle
R¹ a la signification indiquée précédemment ;
en présence d'un composé de formule (IV) 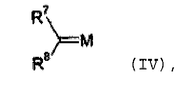 dans laquelle
R⁷, R⁸ sont choisis indépendamment l'un de l'autre parmi H, des groupes alkyle en C₁-C₁₂, cycloalkyle en C₃-C₈, aryle en C₆-C₁₈, arylalkyle en C₇-C₁₉ ou alkylaryle en C₇-C₁₉ ;
M est choisi parmi O, S, Se, NH, NR' et OR', R' ayant les significations définies plus haut.

2. Procédé selon la revendication 1, **caractérisé en ce que** le dérivé d'acide acrylique 2-acylé de formule (II) est choisi dans le groupe constitué par le (2-éthoxyméthylène)-4,4-difluorométhylacétoacétate d'éthyle, le (2-éthoxyméthylène)-4,4,4-trifluorométhyl-acétoacétate d'éthyle, l'éthyl-(2-éthoxyméthylène)-4,4,4-trifluorométhylacétonitrile.

3. Procédé selon la revendication 1 ou 2, **caractérisé en ce que** la N-alkyl-hydrazine de formule (III) est choisie dans le groupe constitué par la monométhylhydrazine, la monoéthylhydrazine, la phénylhydrazine.

4. Procédé selon l'une quelconque des revendications 1 à 3, **caractérisé en ce que** le composé de formule (IV) est choisi dans le groupe constitué par l'acétone, la pinacolone, le benzaldéhyde, la benzophénone.

5. Procédé selon l'une quelconque des revendications 1 à 4, **caractérisé en ce qu'**on utilise comme dérivé d'acide acrylique de formule (II) le (2-éthoxyméthylène)-4,4-difluorométhylacétoacétate d'éthyle, comme hydrazine de formule (III) la méthylhydrazine et comme composé de formule (IV) l'acétone.
